# EUROPEAN PATENT APPLICATION

(11) **EP 4 331 544 A1**
(43) Date of publication of application: **06.03.2024**
(21) Application number: 22811229.8
(22) Date of filing: 18.05.2022
(51) Int. Cl.: A61F 13/15, A61F 13/496, A61F 13/51

(54) **METHOD OF MANUFACTURING ARTICLE OF CLOTHING AND ARTICLE OF CLOTHING**

(30) Priority: 24.05.2021 JP 2021086974
(71) Applicant: Zuiko Corporation, Ibaraki-shi Osaka 5670082 (JP)
(72) Inventor: TANAKA, Yasutaka, Ibaraki-shi, Osaka 567-0082 (JP)
(74) Representative: Hasegawa, Kan
(86) International application number: PCT/JP2022/020738
(87) International publication number: WO 2022/249957

(57) **Abstract**

The present invention provides a method of manufacturing an wearing article (100) including: a front waist part (11) which is worn on a front abdominal side of a wearer and which includes a left-front end (11L) and a right-front end (11R); and a rear waist part (12) which is worn on a back part side of the wearer and which includes a left-rear end (12L) and a right-rear end (12R) forming, together with the left-front end (11L) and a right-front end (11R), a left-side sealing portion (13L) and a right-side sealing portion (13R). In the method, the left-side sealing portion (13L) in a layered state in which a fiber-supplemented sheet (2) formed from a nonwoven fabric is layered on an elastic fiber sheet (1) is formed in at least a portion of the article, the right-side sealing portion (13R) in the layered state is formed in at least a portion of the article, and, in at least a portion of the elastic fiber sheet (1), the fiber-supplemented sheet (2) is not layered such that airflow is allowed to pass through the elastic fiber sheet (1).

## Description

### Technical Field

The present invention relates to a method of manufacturing an wearing article and the wearing article.

### Background Art

A known wearing article is worn on a lower abdomen of a wearer such as an underpants type diaper, for example. An example of a sheet of clothing used for the wearing article includes an elastic sheet. For example, Patent Literature 1 discloses a stretchable nonwoven fabric laminate as an elastic sheet. The stretchable nonwoven fabric laminate is obtained by stretching a nonwoven fabric laminate. Specifically, the stretchable nonwoven fabric laminate is formed by stretching a nonwoven fabric laminate in which an elastic nonwoven fabric and a mixed fiber spunbond nonwoven fabric are laminated. The elastic nonwoven fabric has elasticity, and is elastically recovered when stress is released after stretching. On the other hand, the mixed fiber spunbond nonwoven fabric is extended by drawing, but does not elastically recover even when the stress is released. The elastic sheet described in Patent Literature 1 is applicable to an underpants type wearing article.

However, in a case where the elastic sheet described in Patent Literature 1 is applied to an underpants type wearing article, it is difficult to secure the joint strength of the elastic sheet in a portion from the outside of the groin to the waist of the wearer.

Specifically, the underpants type wearing article includes a front waist part covering the front abdominal part of the wearer and a rear waist part covering the back part of the wearer. The front waist part and the rear waist part are joined to each other at a side sealing portion corresponding to a portion from the outside of the groin to the waist of the wearer. When the underpants type of wearing article is spread in a direction where the front waist part and the rear waist part thus joined are away from each other, stress concentration occurs at the side sealing portion. Therefore, the side sealing portion may have peeling, breakage, or the like of the front waist part and the rear waist part. Therefore, it is necessary to secure the joint strength between the front waist part and the rear waist part in the side sealing portion. However, when the elastic sheet described in Patent Literature 1 is applied to at least one waist part of the front waist part and the rear waist part, it is difficult to secure the joint strength. This is because the waist part applied with the elastic sheet described in Patent Literature 1 has a reduced fiber density due to drawing in the manufacturing process. That is, since the waist part has a small fiber amount per unit area that can be involved in joining, it is difficult to secure the joint strength of the waist part at the side sealing portion. Therefore, the waist part may be broken, peeled, or the like.

### Citation List

### Patent Literature

Patent Literature 1: JP 2019-70221 A

### Summary of Invention

The present invention has been made in view of the above circumstances, and an object of the present invention is to provide a method of manufacturing a wearing article that can secure a joint strength of a side sealing portion, and the wearing article.

A method of manufacturing a wearing article for solving the above problems is a method of manufacturing a wearing article that covers a front abdominal part, a crotch, and a back part of a wearer, the wearing article including a front waist part including a left-front end and a right-front end in an article width direction along a waist of the wearer and covering the front abdominal part of the wearer, and a rear waist part including a left-rear end and a right-rear end in the article width direction forming a left-side sealing portion and a right-side sealing portion, respectively, by being joined to an inner side surface in a state of being layered on the inner side surface of each of the left-front end and the right-front end and covering the back part of the wearer, the rear waist part being coupled to the front waist part at a portion positioned in the crotch of the wearer, the method including: a preparation step of preparing an elastic fiber sheet formed of a nonwoven fabric including an elastic fiber formed of a fiber having elasticity, and an extensible stretching fiber having elasticity smaller than the elasticity of the elastic fiber, the nonwoven fabric subjected to drawing processing; a left-side sealing portion formation step of forming the left-side sealing portion by superimposing and joining the left-front end and the left-rear end of each wearing article in a layered state where a fiber-supplemented sheet formed of a nonwoven fabric is layered on the elastic fiber sheet in at least a part of a portion that is a formation target of the left-side sealing portion; a right-side sealing portion formation step of forming the right-side sealing portion by superimposing and joining the right-front end and the right-rear end of each wearing article in the layered state in at least a part of a portion that is a formation target of the right-side sealing portion; and a clipping step of clipping the front waist part and the rear waist part from the elastic fiber sheet, in which the fiber-supplemented sheet is not layered on the elastic fiber sheet in at least a part of the elastic fiber sheet after the left-side sealing portion formation step and the right-side sealing portion formation step, and airflow through the elastic fiber sheet is allowed.

a wearing article for solving the above problem is a wearing article that covers a front abdominal part, a crotch, and a back part of a wearer, the wearing article including: a front waist part including a left-front end and a right-front end in an article width direction along a waist of the wearer and covering the front abdominal part of the wearer; and a rear waist part including a left-rear end and a right-rear end in the article width direction forming a left-side sealing portion and a right-side sealing portion, respectively, by being joined to an inner side surface in a state of being layered on the inner side surface of each of the left-front end and the right-front end and covering the back part of the wearer, the rear waist part being coupled to the front waist part at a portion positioned in the crotch of the wearer, in which the front waist part and the rear waist part include an elastic fiber sheet formed of a nonwoven fabric including an elastic fiber formed of a fiber having elasticity, and an extensible stretching fiber having elasticity smaller than the elasticity of the elastic fiber, the nonwoven fabric subjected to drawing processing, in the left-side sealing portion, in at least a part of the left-side sealing portion, the left-front end and the left-rear end of each wearing article are superimposed and joined in a layered state where a fiber-supplemented sheet formed of a nonwoven fabric is layered on the elastic fiber sheet, in the right-side sealing portion, in at least a part of the right-side sealing portion, the right-front end and the right-rear end of each wearing article are superimposed and joined in the layered state, and the fiber-supplemented sheet is not layered on the elastic fiber sheet in at least a part of the elastic fiber sheet, and airflow through the elastic fiber sheet is allowed.

According to the present invention, it is possible to provide a method of manufacturing a wearing article that can secure the joint strength of the side sealing portion, and the wearing article.

### Brief Description of Drawings

FIG. 1 is a front view of a wearing article according to an embodiment of the present invention in a folded state.
FIG. 2 is a perspective view of the wearing article of FIG. 1.
FIG. 3 is a developed view of a developed state of the wearing article of FIG. 1 as viewed from an absorbent main body side.
FIG. 4 is a developed view of the developed view of FIG. 3 as viewed from a back side.
FIG. 5 is a cross-sectional view taken along line V-V of FIG. 3.
FIG. 6 is a schematic view illustrating a flow of a method of manufacturing the wearing article of FIG. 1.
FIG. 7 is an enlarged view of a portion where a sheet layered body in FIG. 6 is separated in a sheet width direction.
FIG. 8 is an enlarged view of a portion where the wearing article in FIG. 6 is cut.

### Description of Embodiments

An embodiment of the present invention will be described below with reference to the accompanying drawings. Note that the following embodiment is an example embodying the present invention, and is not intended to limit the technical scope of the present invention.

### <Wearing Article>

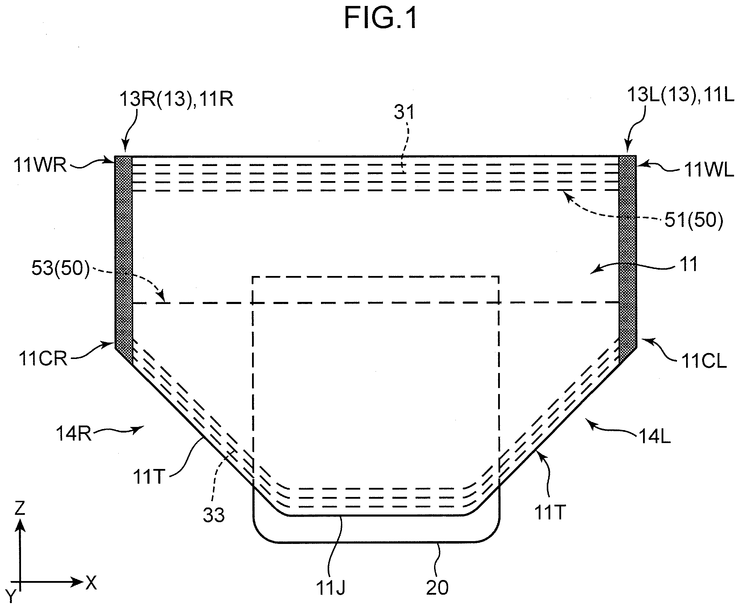

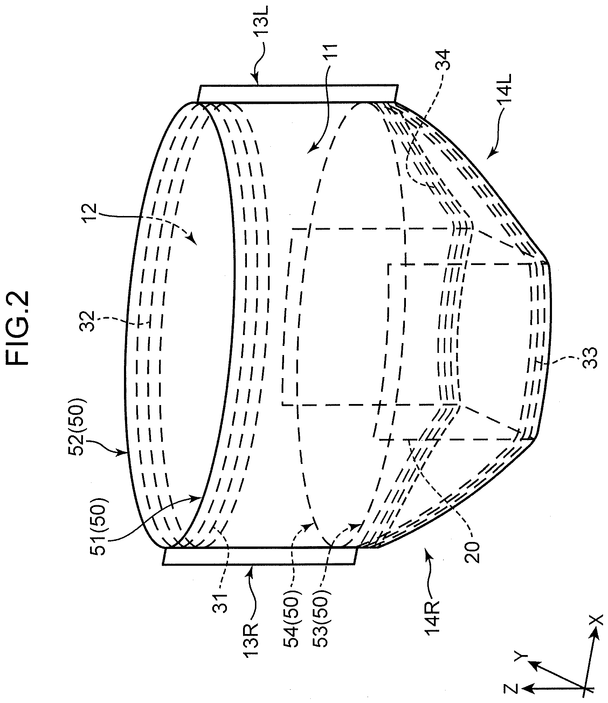

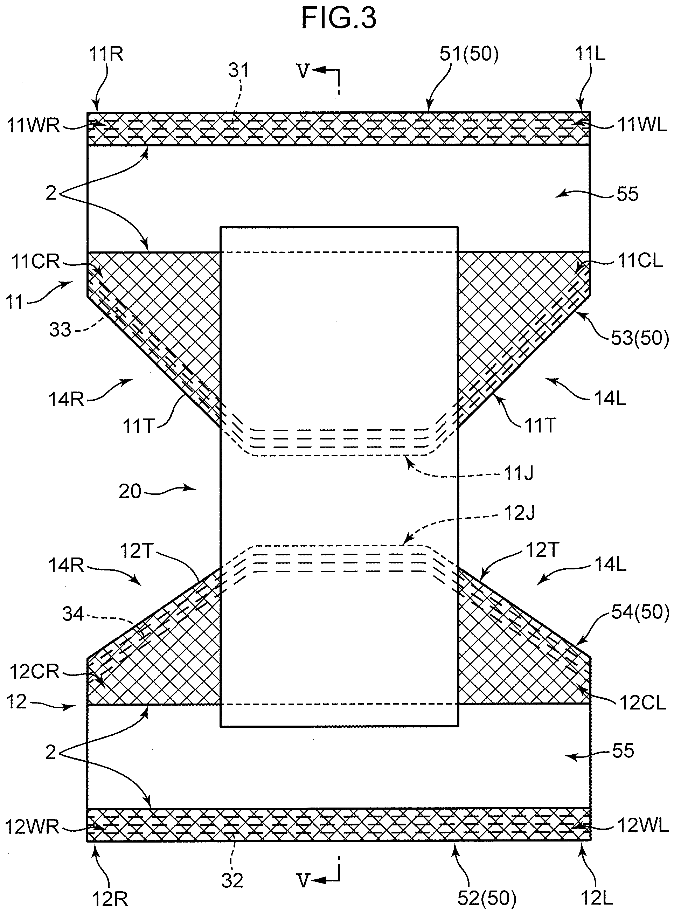

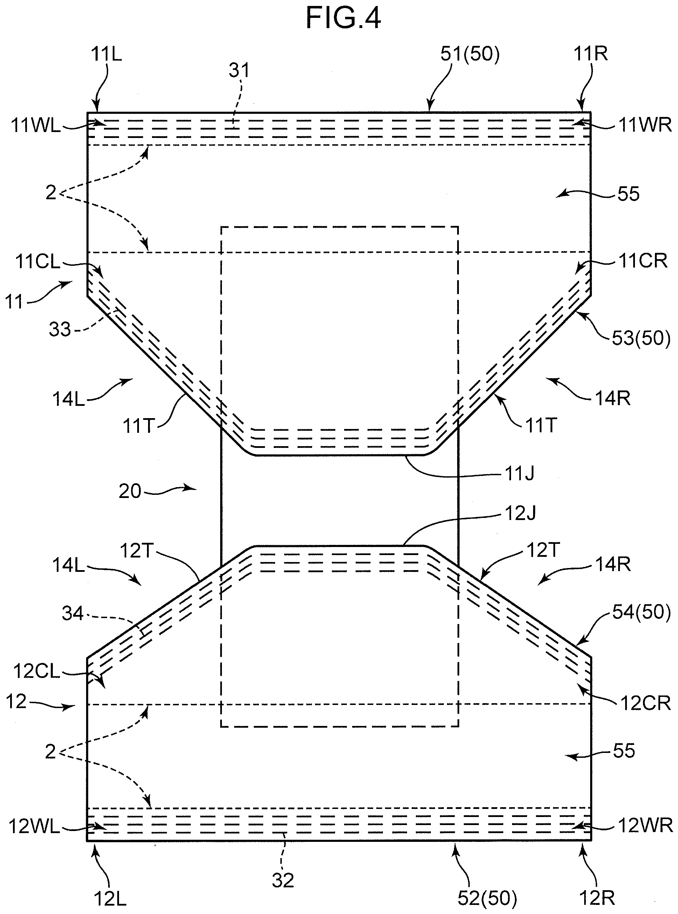

A wearing article according to an embodiment of the present invention will be described below. FIGS. 1 and 2 are views illustrating the wearing article according to the embodiment of the present invention, and FIGS. 3 and 4 are views illustrating the wearing article in a developed manner illustrated in FIGS. 1 and 2 in order to facilitate the description of the wearing article.

As illustrated in FIGS. 1 and 2, a wearing article 100 includes, in a state of being worn by a wearer, a portion extending in an article width direction, which is a direction along the waist of the wearer, and covering the back part and the front abdominal part, and a portion extending in an article front-rear direction orthogonal to the article width direction and covering the crotch of the wearer.

In the wearing article 100, the article width direction is an X direction in FIGS. 1 and 2, and coincides with a developed width direction in FIGS. 3 and 4 (left-right direction in FIGS. 3 and 4). The article front-rear direction is a Y direction in FIGS. 1 and 2. A Z direction in FIGS. 1 and 2 is orthogonal to the X direction and the Y direction.

As illustrated in FIGS. 1 and 2, the wearing article 100 includes a front waist part 11 covering at least the front abdominal part of the wearer and a rear waist part 12 covering at least the back part of the wearer, an absorbent main body 20 that absorbs a body fluid of the wearer, and a plurality of elastic members 31 to 34 imparting elasticity to the waist parts 11 and 12.

In this wearing article 100, the front waist part 11 and the rear waist part 12 are coupled in the article front-rear direction via the absorbent main body 20 covering a part of the crotch of the wearer. The front waist part 11 and the rear waist part 12 are joined at a side sealing portion 13 positioned in a portion from an outside of the groin to the waist of the wearer. Specifically, the front waist part 11 and the rear waist part 12 are joined at a left-side sealing portion 13L on a left side of both ends in the article width direction of the front waist part 11 and the rear waist part 12, and at a right-side sealing portion 13R on a right side of the both ends. The left-side sealing portion 13L extends in a left seal extending direction. The right-side sealing portion 13R extends in a right seal extending direction. The left seal extending direction and the right seal extending direction coincide with the Z direction.

In this wearing article 100, the ends on the waist side of the wearer of the front waist part 11 and the rear waist part 12 are opened so that the lower abdomen of the wearer can be inserted. On the other hand, a left leg hole 14L and a right leg hole 14R that are linear in front view are formed at an end on the crotch side of the wearer in the wearing article 100. The left leg hole 14L and the right leg hole 14R are openings into which the left leg and the right leg of the wearer can be inserted and which are formed along the circumference of the leg. The left leg hole 14L and the right leg hole 14R may be curved in front view.

As illustrated in FIGS. 3 and 4, the absorbent main body 20 has a rectangular shape, and is disposed in a state where a longitudinal direction thereof is along a developed up-down direction in FIGS. 3 and 4 (up-down direction illustrated in FIGS. 3 and 4). The absorbent main body 20 has a core for absorbing the body fluid of the wearer. The core includes, for example, a super absorbent polymer (SAP) such as sodium polyacrylate, an absorbent material such as fluff pulp etc., and a liquid-permeable top sheet and a liquid-impermeable back sheet (not illustrated) sandwiching them.

The front waist part 11 and the rear waist part 12 are configured to secure the joint strength of the left-side sealing portion 13L and the right-side sealing portion 13R while securing the elasticity and air permeability of the wearing article 100. Specifically, the front waist part 11 and the rear waist part 12 include an elastic fiber sheet 1 and a fiber-supplemented sheet 2 layered and joined only to a plurality of predetermined sheet layering regions 50 of the elastic fiber sheet 1. The plurality of sheet layering regions 50 are regions set as regions in which the fiber-supplemented sheet 2 is layered on the elastic fiber sheet 1.

The elastic fiber sheet 1 is a nonwoven fabric having a relatively low fiber density, and is excellent in elasticity and air permeability. By adopting the elastic fiber sheet 1 for the front waist part 11 and the rear waist part 12, the front waist part 11 and the rear waist part 12 are given elasticity and air permeability. The elastic fiber sheet 1 is formed of a nonwoven fabric including an elastic fiber formed of a fiber having elasticity and an extensible stretching fiber having elasticity smaller than the elasticity of the elastic fiber, the nonwoven fabric subjected to elastic processing.

As the elastic fiber, for example, thermoplastic elastomers such as olefin-based elastomers, styrene-based elastomers, polyurethane-based elastomers, polyester-based elastomers, and polyamide-based elastomers can be used. Other than that, elastic fibers made from a propylene-ethylene copolymer, natural rubber, or the like as a raw material may be used.

The stretching fiber has elasticity smaller than that of the elastic fiber and is inherently poor in elasticity, but is given a function of stretching by drawing. As the stretching fiber, for example, polyethylene, polypropylene, polyester such as polyethylene terephthalate, polyamide, or the like can be used.

Examples of a method of manufacturing a fiber in which the elastic fiber and the stretching fiber are combined include a method by layering a layer containing the elastic fiber and a layer containing the stretching fiber, and a method by mixing the elastic fiber and the stretching fiber.

On the other hand, the fiber-supplemented sheet 2 is a nonwoven fabric. As the fiber constituting the fiber-supplemented sheet 2, for example, thermoplastic resins such as polyolefin-based resins such as polyethylene and polypropylene, polyester-based resins such as polyethylene terephthalate and polybutylene terephthalate, polyamide-based resins such as nylon, vinyl-based resins such as polystyrene and polyvinyl chloride, acrylonitrile-based resins such as acrylic, methacrylic resins, and vinylidene resins can be used. The fiber density of the fiber-supplemented sheet 2 is not particularly limited, but by making the fiber density larger than the fiber density of the elastic fiber sheet 1, the adhesive strength described later can be reliably improved.

The configuration of each part of the front waist part 11 and the rear waist part 12 will be further described.

The front waist part 11 is a sheet covering a part of the crotch on the front abdominal side in addition to the front abdominal part of the wearer in the present embodiment. The front waist part 11 includes a portion covering the front abdominal part of the wearer, a portion covering both sides of the wearer, and a portion covering a part of the crotch on the front abdominal side of the wearer in accordance with the shapes of the front abdominal part and the crotch on the front abdominal side.

The front waist part 11 has a left-front end 11L on the left side in the article width direction and a right-front end 11R on the right side in the article width direction. Furthermore, the front waist part 11 includes a front waist side extending in the article width direction between upper ends of both the front ends 11L and 11R, a pair of front crotch inclined sides 11T extending downward from lower ends of both the front ends 11L and 11R in a direction approaching each other, and a front coupling side 11J extending in the article width direction so as to couple the front crotch inclined sides 11T to each other.

The pair of front crotch inclined sides 11T respectively form a part of the left leg hole 14L and a part of the right leg hole 14R. Specifically, the pair of front crotch inclined sides 11T are inclined so as to approach the portion where the absorbent main body 20 is disposed from lower ends (a left-front crotch end 11 CL and a right-front crotch end 11CR) of the left-front end 11L and the right-front end 11R, respectively.

The fiber-supplemented sheet 2 is layered on an inner side surface of the elastic fiber sheet 1 of the front waist part 11 in each of the plurality of sheet layering regions 50. The plurality of sheet layering regions 50 are regions where the fiber-supplemented sheet 2 is layered on the elastic fiber sheet 1. In the present embodiment, the plurality of sheet layering regions 50 of the front waist part 11 include a front waist region 51 and a front crotch region 53 (see FIG. 3) set at intervals in the Z direction illustrated in FIGS. 1 and 2.

The front waist region 51 is a region extending along the front waist side of the front waist part 11. Specifically, the front waist region 51 extends from a left-front waist end 11WL to a right-front waist end 11WR in the article width direction illustrated in FIG. 1. The left-front waist end 11 WL is an end close to the waist of the wearer of both ends in the left seal extending direction of the left-front end 11L. The right-front waist end 11WR is an end close to the waist of the wearer of both ends in the right seal extending direction of the right-front end 11R.

The front crotch region 53 is a region set at an end close to the crotch of the front waist part 11. Specifically, the front crotch region 53 is a region including the front coupling side 11J, the both front crotch inclined sides 11T, and the ends (the left-front crotch end 11CL and the right-front crotch end 11CR) close to the crotch of both the front ends 11L and 11R. The left-front crotch end 11CL is an end close to the crotch of the wearer of both ends in the left seal extending direction of the left-front end 11L. The right-front crotch end 11CR is an end close to the crotch of the wearer of both ends in the right seal extending direction of the right-front end 11R.

A non-disposition region 55 is set between the front waist region 51 and the front crotch region 53. The non-disposition region 55 is a region where the fiber-supplemented sheet 2 is not layered on the elastic fiber sheet 1 and airflow through the elastic fiber sheet 1 is allowed.

On the other hand, the rear waist part 12 is a sheet covering a part of the crotch on the back part side in addition to the back part of the wearer in the present embodiment. In the present embodiment, the rear waist part 12 has the same shape and the same size as those of the front waist part 11, and has the same sheet configuration. Specifically, the rear waist part 12 includes a portion covering the back part of the wearer, a portion covering the both sides of the wearer, and a portion covering a part of the crotch on the back part side of the wearer in accordance with the shapes of the back part and the crotch on back part side.

The rear waist part 12 has a left-rear end 12L on the left side in the article width direction and a right-rear end 12R on the right side in the article width direction. Furthermore, the rear waist part 12 includes a rear waist side extending in the article width direction between upper ends of the rear ends 12L and 12R, a pair of rear crotch inclined sides 12T extending downward from lower ends of both the rear ends 12L and 12R in a direction approaching each other, and a rear coupling side 12J extending in the article width direction so as to couple the rear crotch inclined sides 12T to each other.

The pair of rear crotch inclined sides 12T respectively form a part of the left leg hole 14L and a part of the right leg hole 14R. Specifically, the pair of rear crotch inclined sides 12T are inclined so as to approach the portion where the absorbent main body 20 is disposed from lower ends (a left-rear crotch end 12CL and a right-rear crotch end 12CR) of the left-rear end 12L and the right-rear end 12R, respectively.

The fiber-supplemented sheet 2 is layered on an inner side surface of the elastic fiber sheet 1 of the rear waist part 12 in each of the plurality of sheet layering regions 50. In the present embodiment, the plurality of sheet layering regions 50 of the rear waist part 12 include a rear waist region 52 and a rear crotch region 54 set at intervals in the Z direction illustrated in FIGS. 1 and 2. The rear waist region 52 and the rear crotch region 54 are regions opposing the front waist region 51 and the front crotch region 53 (see FIG. 3), respectively, when the front waist part 11 and the rear waist part 12 are joined.

The rear waist region 52 is a region extending along the rear waist side of the rear waist part 12. Specifically, the rear waist region 52 extends from a left-rear waist end 12WL to a right-rear waist end 12WR in the article width direction illustrated in FIG. 1. The left-rear waist end 12WL is an end close to the waist of the wearer of both ends in the left seal extending direction of the left-rear end 12L. The right-rear waist end 12WR is an end close to the waist of the wearer of both ends in the right seal extending direction of the right-rear end 12R.

The rear crotch region 54 is a region set at an end close to the crotch of the rear waist part 12. Specifically, the rear crotch region 54 is a region including the rear coupling side 12J, the both rear crotch inclined sides 12T, and the ends (the left-rear crotch end 12CL and the right-rear crotch end 12CR) close to the crotch of both the rear ends 12L and 12R. The left-rear crotch end 12CL is an end close to the crotch of the wearer of both ends in the left seal extending direction of the left-rear end 12L. The right-rear crotch end 12CR is an end close to the crotch of the wearer of both ends in the right seal extending direction of the right-rear end 12R.

The non-disposition region 55 is set between the rear waist region 52 and the rear crotch region 54. The non-disposition region 55 is a region where the fiber-supplemented sheet 2 is not layered on the elastic fiber sheet 1 and airflow through the elastic fiber sheet 1 is allowed.

The front waist part 11 and the rear waist part 12 are joined at each of the left-side sealing portion 13L and the right-side sealing portion 13R. In the present embodiment, both ends in the left seal extending direction of the left-side sealing portion 13L and both ends in the right seal extending direction of the right-side sealing portion 13R are in a layered state where the fiber-supplemented sheet 2 is layered on the elastic fiber sheet 1, and the elastic fiber sheet 1 and the fiber-supplemented sheet 2 are joined by thermal welding in the layered state. Therefore, the joint strength can be secured at the both ends of the left-side sealing portion 13L and the both ends of the right-side sealing portion 13R.

Specifically, the elastic fiber sheet 1 and the fiber-supplemented sheet 2 are in a layered state in the front waist region 51 and the front crotch region 53 of the front waist part 11. Due to this, the elastic fiber sheet 1 and the fiber-supplemented sheet 2 are in a layered state at both ends in the left seal extending direction of the left-front end 11L and at both ends in the right seal extending direction of the right-front end 11R.

Furthermore, the elastic fiber sheet 1 and the fiber-supplemented sheet 2 are in a layered state in the rear waist region 52 and the rear crotch region 54 of the rear waist part 12. Due to this, the elastic fiber sheet 1 and the fiber-supplemented sheet 2 are in a layered state at both ends in the left seal extending direction of the left-rear end 12L and at both ends in the right seal extending direction of the right-rear end 12R.

That is, in the left-side sealing portion 13L, the both ends in the left seal extending direction of the left-front end 11L and the both ends in the left seal extending direction of the left-rear end 12L are joined in the layered state. Similarly, in the right-side sealing portion 13R, the both ends in the right seal extending direction of the right-front end 11R and the both ends in the right seal extending direction of the right-rear end 12R are joined in the layered state. Specifically, the elastic fiber sheet 1 and the fiber-supplemented sheet 2 are layered in a state where two layers of the fiber-supplemented sheet 2 are interposed between the elastic fiber sheet 1 and the elastic fiber sheet 1. Therefore, although the fiber density of the elastic fiber sheet 1 is low, the fiber density can be supplemented by the fiber-supplemented sheet 2 in both the side sealing portions 13L and 13R. The fiber amount per unit area of the portion where the elastic fiber sheet 1 and the fiber-supplemented sheet 2 are integrally joined in a layered state becomes larger than the fiber amount per unit area of only the elastic fiber sheet 1, thereby the fiber amount involved in joining in the portion increases, and thus a sufficient joint strength can be secured. Therefore, the joint strength can be secured at the both ends of the left-side sealing portion 13L and the both ends of the right-side sealing portion 13R.

The both ends of the left-side sealing portion 13L are portions where stress concentration particularly occurs when the front waist part 11 and the rear waist part 12 in a state of being joined to each other are expanded in a direction away from each other. By ensuring the joint strength of the both ends as described above, it is possible to suppress the left-side sealing portion 13L from being unintentionally torn from the both ends. Similarly, it is possible to suppress the right-side sealing portion 13R from being unintentionally torn from the both ends of the right-side sealing portion 13R, which are portions where stress concentration particularly occurs.

Note that when the fiber density of the fiber-supplemented sheet 2 is larger than the fiber density of the elastic fiber sheet 1, the fiber amount per unit area of the portion where the elastic fiber sheet 1 and the fiber-supplemented sheet 2 are integrally joined in a layered state becomes reliably larger than the fiber amount per unit area of only the elastic fiber sheet 1, whereby a sufficient joint strength in the portion can be secured.

On the other hand, in the front waist part 11, since the fiber-supplemented sheet 2 is not layered on the elastic fiber sheet 1 in the non-disposition region 55, airflow through the elastic fiber sheet 1 is secured. Similarly, in the rear waist part 12, since the fiber-supplemented sheet 2 is not layered on the elastic fiber sheet 1 in the non-disposition region 55, airflow through the elastic fiber sheet 1 is secured.

As described above, in the wearing article 100 in which the front waist part 11 and the rear waist part 12 are joined, although the fiber density of the elastic fiber sheet 1 is low, the joint strength of the left-side sealing portion 13L and the right-side sealing portion 13R can be secured and the air permeability of the elastic fiber sheet 1 can be secured.

The plurality of elastic members 31 to 34 impart elasticity to a portion positioned at the waist or the groin of the crotch of the wearer in the wearing article 100. The plurality of elastic members 31 to 34 include a front waist elastic member 31 that imparts elasticity to the front abdominal side of the waist of the wearer, a rear waist elastic member 32 that imparts elasticity to the back part side of the waist of the wearer, a front crotch elastic member 33 that imparts elasticity to the front abdominal side of the groin of the wearer, and a rear crotch elastic member 34 that imparts elasticity to the back part side of the groin of the wearer.

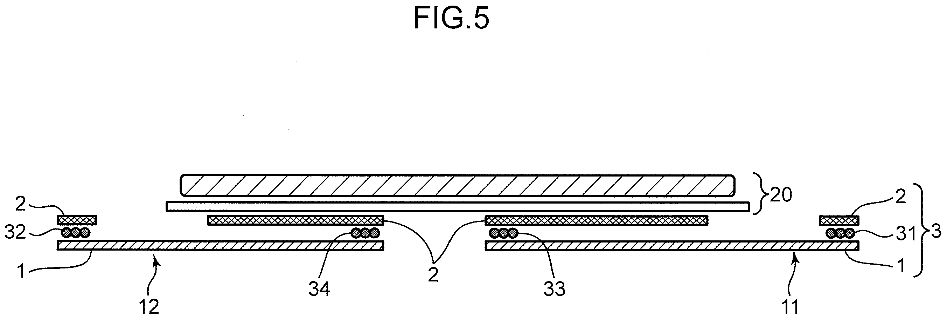

The front waist elastic member 31 is provided in a state of being elastic in the article width direction between the elastic fiber sheet 1 and the fiber-supplemented sheet 2 (FIG. 5) in the front waist region 51. In the present embodiment, a plurality of the front waist elastic members 31 are disposed at intervals in the Z direction (FIGS. 1 and 2) and extend in the article width direction. The front waist part 11 can be fitted to the waist on the front abdominal side of the wearer by the elastic force of the front waist elastic member 31.

The rear waist elastic member 32 is provided in a state of being elastic in the article width direction between the elastic fiber sheet 1 and the fiber-supplemented sheet 2 (FIG. 5) in the rear waist region 52. In the present embodiment, a plurality of the rear waist elastic members 32 are disposed at intervals in the Z direction (FIGS. 1 and 2) and extend in the article width direction. The rear waist part 12 can be fitted to the waist on the back part side of the wearer by the elastic force of the rear waist elastic member 32.

The front crotch elastic member 33 is provided in a state of being elastic in the article width direction between the elastic fiber sheet 1 and the fiber-supplemented sheet 2 (FIG. 5) in the front crotch region 53. The front waist part 11 can be fitted to the front side of the groin of the wearer by the elastic force of the front crotch elastic member 33.

The rear crotch elastic member 34 is provided in a state of being elastic in the article width direction between the elastic fiber sheet 1 and the fiber-supplemented sheet 2 (FIG. 5) in the rear crotch region 54. The rear waist part 12 can be fitted to the front side of the groin of the wearer by the elastic force of the rear crotch elastic member 34.

The elastic members 31 to 34 are formed of, for example, a material such as polyurethane, natural rubber, or thermoplastic resin.

### <Method of Manufacturing Wearing article>

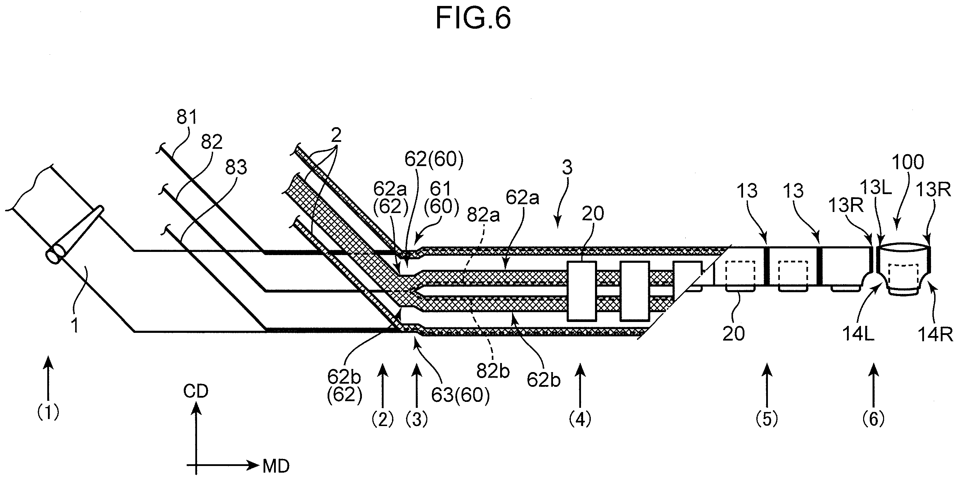

Next, a method of manufacturing the wearing article 100 will be described. FIG. 6 is a schematic view illustrating the flow of the method of manufacturing the wearing article of FIG. 1. The method of manufacturing the wearing article 100 includes (1) a first sheet preparation step, (2) a second sheet preparation step, (3) a separation step, (4) an absorbent main body disposition step, (5) a side sealing portion formation step, and (6) a cutting step.

### (1) First sheet preparation step

In the first sheet preparation step of the present embodiment, the elastic fiber sheet 1 extending in a sheet longitudinal direction corresponding to the article width direction is prepared. The elastic fiber sheet 1 is formed of a nonwoven fabric including an elastic fiber formed of a fiber having elasticity and an extensible stretching fiber having elasticity smaller than the elasticity of the elastic fiber, the nonwoven fabric subjected to drawing processing. This elastic fiber sheet 1 is formed, for example, by layering a layer having the elastic fiber and a layer having the stretching fiber, and then drawing the layer. By this drawing, the layer having the elastic fiber is drawn. On the other hand, the layer having the stretching fiber is extended by being plastically deformed by drawing. When the stress is released in the drawn nonwoven fabric, the layer having the elastic fiber is elastically recovered, and on the other hand, the layer having the stretching fiber is not elastically recovered but compressed by the elasticity of the elastic fiber in a state of being extended. The elastic fiber sheet 1 thus obtained has a relatively low fiber density and is excellent in elasticity and air permeability.

For the drawing of the nonwoven fabric mentioned above, for example, gear drawing performed by passing the nonwoven fabric through between a pair of gear rolls can be adopted. Specifically, each gear roll has a plurality of blades protruding from the outer peripheral surface. By rotating the blades of the pair of gear rolls while meshing with each other as well as passing the nonwoven fabric through between the pair of gear rolls, the nonwoven fabric is drawn. Note that the drawing is not limited to gear drawing, and for example, uniaxial drawing, biaxial drawing, partial drawing, overall drawing, or the like can be adopted.

Other than that, the elastic fiber sheet 1 can be formed by mixing the elastic fiber and the stretching fiber. For example, a thermoplastic elastomer that is a material of elastic fibers and a thermoplastic resin that is a material of stretching fibers are molten in a state of being mixed. The nonwoven fabric is formed by depositing fibers obtained by melt-spinning a material in a molten state. The elastic fiber sheet 1 can be formed by drawing the nonwoven fabric as described above.

In the method according to this embodiment, an elastic fiber sheet extending in the sheet longitudinal direction and having a width dimension in the sheet width direction orthogonal to the sheet longitudinal direction is prepared as the elastic fiber sheet 1.

The width dimension of the elastic fiber sheet 1 corresponds to a dimension obtained by adding a dimension from the front waist side to the front coupling side 11J of the front waist part 11 and a dimension from the coupling side 12J to the rear waist side of the rear waist part 12. That is, a predetermined width dimension in the sheet width direction has a dimension where the elastic fiber sheet 1 can be cut in a state where the region where the front waist part 11 is formed and the region where the rear waist part 12 is formed are arranged side by side in the sheet width direction.

The length dimension of the elastic fiber sheet 1 is set to a dimension where a portion that becomes a plurality of the wearing articles 100 can be clipped by splitting the elastic fiber sheet 1 in the sheet longitudinal direction as in "(6) Cutting step" described later. The portion that becomes each of the wearing articles 100 includes only a portion constituting each of one front waist part 11 and one rear waist part 12.

A plurality of layering regions 60 extending in the sheet longitudinal direction are set in the elastic fiber sheet 1. The plurality of layering regions 60 are regions set as regions in which the fiber-supplemented sheet 2 extending in the sheet longitudinal direction is layered on the elastic fiber sheet 1. The plurality of layering regions 60 are set to be arranged side by side at intervals in the sheet width direction. Specifically, in the present embodiment, as illustrated in FIG. 6, the plurality of layering regions 60 include a first end region 61, an intermediate region 62, and a second end region 63. The first end region 61, the intermediate region 62, and the second end region 63 extend in the sheet longitudinal direction. The first end region 61 is a region along one of the both ends in the sheet width direction of the elastic fiber sheet 1. The second end region 63 is a region along the other end of the both ends in the sheet width direction of the elastic fiber sheet 1. The intermediate region 62 is a region between the first end region 61 and the second end region 63 in the elastic fiber sheet 1.

The intermediate region 62 includes a first intermediate region 62a and a second intermediate region 62b separated in the sheet width direction in "(3) Separation step" described later. The first intermediate region 62a is a region on a side close to the first end region 61 in the intermediate region 62, and the second intermediate region 62b is a region on a side close to the second end region 63 in the intermediate region 62.

The first end region 61 and the first intermediate region 62a are included in a region where the front waist part 11 is formed. The second end region 63 and the second intermediate region 62b are included in a region where the rear waist part 12 is formed.

The plurality of layering regions 60 of the elastic fiber sheet 1 correspond to the plurality of sheet layering regions 50 of the wearing article 100 continuous in the sheet longitudinal direction. Specifically, the first end region 61, the first intermediate region 62a, the second intermediate region 62b, and the second end region 63 correspond to the front waist region 51, the front crotch region 53, the rear crotch region 54, and the rear waist region 52, respectively, of the wearing article 100 that are each continuous in the sheet longitudinal direction. A region between the first end region 61 and the first intermediate region 62a and a region between the second end region 63 and the second intermediate region 62b correspond to the non-disposition region 55 continuous in the sheet longitudinal direction.

The plurality of layering regions 60 include two pairs of opposable regions. The opposable region is a region opposing to each other when sheet layered bodies 3 are superimposed in the sheet width direction in "(5) Side sealing portion formation step" described later. The sheet layered body 3 is a sheet in which the fiber-supplemented sheet 2 is layered on the elastic fiber sheet 1. In the present embodiment, the first end region 61 and the second end region 63 correspond to a pair of the opposable regions, and the first intermediate region 62a and the second intermediate region 62b correspond to a pair of the opposable regions.
"(1) First sheet preparation step" of the present embodiment is included in the "preparation step of preparing an elastic fiber sheet" in the claims of the present invention.
(2) Second sheet preparation step

In the second sheet preparation step, the sheet layered body 3 in which the elastic fiber sheet 1, a plurality of elastic members 81 to 83, and a plurality of the fiber-supplemented sheets 2 are layered is formed.

Specifically, in the second sheet preparation step, the elastic fiber sheet 1 is conveyed in a machine direction MD parallel to the sheet longitudinal direction thereof. The sheet longitudinal direction coincides with the article width direction of the wearing article 100 illustrated in FIGS. 1 and 2. A crossing direction CD illustrated in FIG. 6 is a direction orthogonal to the machine direction MD.

In the second sheet preparation step, in a state where the elastic fiber sheet 1 is conveyed, the plurality of elastic members 81 to 83 are disposed on the plurality of layering regions 60 of the elastic fiber sheet 1 while being conveyed in a state of being extended in the sheet longitudinal direction.

Specifically, a plurality of first end elastic members 81 are disposed on the first end region 61 of the elastic fiber sheet 1 in a state of being disposed at intervals in the sheet width direction. A plurality of second end elastic members 83 are disposed on the second end region 63 of the elastic fiber sheet 1 in a state of being disposed at intervals in the sheet width direction. Furthermore, a plurality of intermediate elastic members 82 are disposed on the intermediate region 62 of the elastic fiber sheet 1 in a state of being disposed at intervals in the sheet width direction. Specifically, in the intermediate region 62, a first intermediate elastic member 82a is disposed in a region on the first end region 61 side with reference to the cutting line to be cut in the separation step (3), that is, on the first intermediate region 62a. In the intermediate region 62, a second intermediate elastic member 82b is disposed in a region on the second end region 63 side with reference to the cutting line, that is, on the second intermediate region 62b.

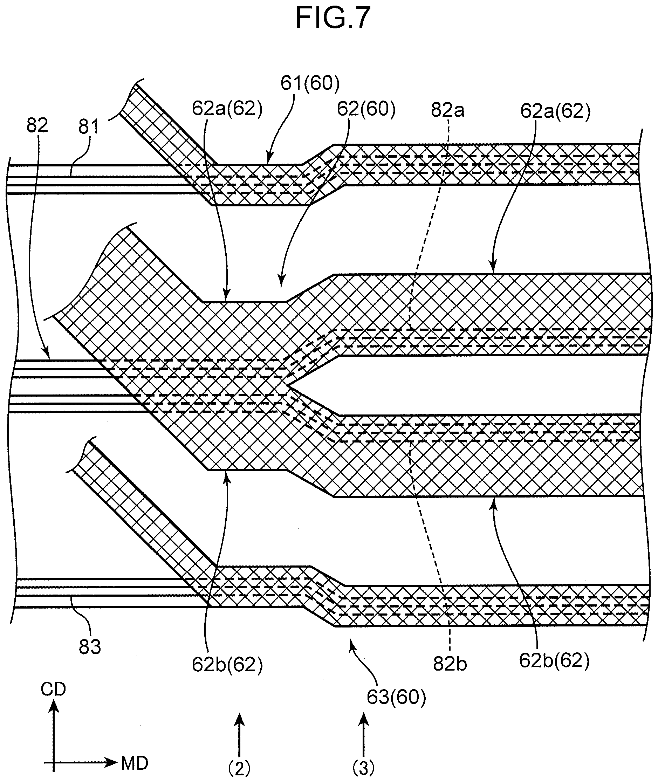

Note that the plurality of intermediate elastic members 82 are drawn linearly in FIGS. 6 and 7, but are actually conveyed along a path along the edges of the left leg hole 14L and the right leg hole 14R.

In the second sheet preparation step, the plurality of fiber-supplemented sheets 2 are joined to the elastic fiber sheet 1 so as to cover the elastic members 81 to 83 from above in each of the first end region 61, the intermediate region 62, and the second end region 63 of the elastic fiber sheet 1 while the fiber-supplemented sheet 2 is conveyed along the sheet longitudinal direction. That is, the elastic fiber sheet 1 and the plurality of fiber-supplemented sheets 2 are joined to each other in a state where the elastic members 81 to 83 are sandwiched between the elastic fiber sheet 1 and the plurality of fiber-supplemented sheets 2. The elastic members 81 to 83 may be joined to at least one of the elastic fiber sheet 1 and the fiber-supplemented sheet 2 sandwiching the elastic members 81 to 83. By joining the elastic fiber sheet 1, the fiber-supplemented sheet 2, and the elastic members 81 to 83, the sheet layered body 3 in which these are layered is formed. The joint means can be adhesion using a hot melt adhesive or welding by heat sealing, but ultrasonic welding is preferable.

"(2) Second sheet preparation step" of the present embodiment is included in the "preparation step of preparing an elastic fiber sheet" in the claims of the present invention. The "preparation step of preparing an elastic fiber sheet" in the claims of the present invention only may include at least "(1) First sheet preparation step" of "(1) First sheet preparation step" and "(2) Second sheet preparation step".

### (3) Separation step

In the separation step, as illustrated in FIGS. 6 and 7, the sheet layered body 3 is cut in the sheet width direction along a cutting line set in advance in a state where the sheet layered body 3 is conveyed in the sheet longitudinal direction. By this cutting, the intermediate region 62 is separated into the first intermediate region 62a and the second intermediate region 62b in the sheet width direction. The region where the front waist part 11 is formed and the region where the rear waist part 12 is formed in the sheet layered body 3 are separated by the cutting.

With this separation, the intermediate elastic member 82 is separated into the first intermediate elastic member 82a and the second intermediate elastic member 82b. Furthermore, in the separation step, the conveyance path of the sheet layered body 3 is changed such that an interval is provided between the region where the front waist part 11 is formed and the region where the rear waist part 12 is formed in the sheet layered body 3.

In "(3) Separation step" of the present embodiment, since the region where the front waist part 11 is formed and the region where the rear waist part 12 is formed are separated in the sheet layered body 3, it corresponds to clipping of a part of the front waist part 11 and a part of the rear waist part 12. Therefore, "(3) Separation step" of the present embodiment is included in the "clipping step of clipping the front waist part and the rear waist part from the elastic fiber sheet" in the claims of the present invention.

### (4) Absorbent main body disposition step

In the absorbent main body disposition step, the absorbent main body 20 is disposed so as to couple the regions separated in "(3) Separation step", that is, the region where the front waist part 11 is formed and the region where the rear waist part 12 is formed to each other, and the absorbent main body 20 is joined to the both regions. In the present embodiment, each absorbent main body 20 is disposed on the sheet layered body 3 so as to cover a region from a position on the first end region 61 side relative to the first intermediate region 62a to a position on the second end region 63 side relative to the second intermediate region 62b of the sheet layered body 3.

### (5) Side sealing portion formation step

The side sealing portion formation step in the present embodiment includes a step of forming the left-side sealing portion 13L and a step of forming the right-side sealing portion 13R of each wearing article 100.

In the side sealing portion formation step, the sheet layered bodies 3 are superimposed by folding the absorbent main body 20 in two in the sheet width direction while conveying the sheet layered body 3 in the sheet longitudinal direction. Due to this, the first end region 61 and the second end region 63 oppose each other, and the first intermediate region 62a and the second intermediate region 62b oppose each other.

In the side sealing portion formation step, in a state where the sheet layered body 3 is superimposed in the sheet width direction, a predetermined length portion along the sheet width direction of the sheet layered body 3 is heated at intervals for each portion forming the wearing article 100 in the sheet longitudinal direction. Due to this, the elastic fiber sheet 1 and the fiber-supplemented sheet 2 in the heated portion are brought into a molten state, and the superimposed sheet layered bodies 3 are joined at the portion. This joined portion forms the side sealing portion 13 of each wearing article 100 in the sheet layered body 3.

Specifically, in the side sealing portion formation step, the side sealing portion 13 that can form the left-side sealing portion 13L of the wearing article 100 on a downstream side and the right-side sealing portion 13R of the wearing article 100 on an upstream side is formed by being cut in the article width direction in the cutting step described later. More specifically, the side sealing portion 13 having a width obtained by adding the width of the left-side sealing portion 13L and the width of the right-side sealing portion 13R in the article width direction is formed. The interval between the continuous side sealing portions 13 corresponds to the dimension in the width direction of the wearing article 100. The "heating" is not only limited to a case where heat is directly applied to the sheet but also includes a case where the sheet is heated by applying ultrasonic vibration to the sheet.

### (6) Cutting step

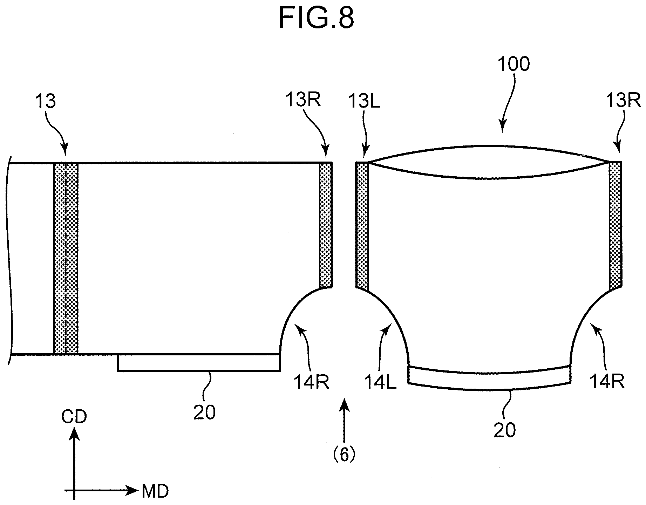

In the cutting step, as illustrated in FIGS. 6 and 8, a continuous body of the portion forming the wearing article 100 is cut so as to be divided in the sheet longitudinal direction for each portion forming the wearing article 100 while the continuous body of the portion forming the wearing article 100 is conveyed in the sheet longitudinal direction in a state where the side sealing portion 13 is coupled. By this, each wearing article 100 including the front waist part 11 and the rear waist part 12 is clipped from the sheet layered body 3. Specifically, the sheet layered body 3 is cut in the sheet width direction at the center position of the sheet longitudinal direction of each of the plurality of side sealing portions 13 formed as described above. Due to this, the side sealing portion 13 is separated into the left-side sealing portion 13L of the wearing article 100 on the downstream side and the right-side sealing portion 13R of the wearing article 100 on the upstream side, and the wearing article 100 on the downstream side is cut from the sheet layered body 3. By sequentially cutting the central position of the side sealing portions 13 in this manner, the wearing articles having the right-side sealing portion 13R and the left-side sealing portion 13L are continuously cut.

"(6) Cutting step" of the present embodiment is included in the "clipping step of clipping the front waist part and the rear waist part from the elastic fiber sheet" in the claims of the present invention because the sheet layered body 3 is cut for each wearing article 100.

In the cutting step, the wearing article 100 is linearly cut so as to further constitute the left leg hole 14L and the right leg hole 14R. The cutting of both the leg holes 14L and 14R may be performed before the absorbent main body disposition step.

According to the method of manufacturing the wearing article 100, the wearing article 100 in which the front waist part 11 and the rear waist part 12 are joined can secure the joint strength of the left-side sealing portion 13L and the right-side sealing portion 13R and can secure air permeability of the elastic fiber sheet 1, although the fiber density of the elastic fiber sheet 1 is low.

Specifically, in each region of the first end region 61, the first intermediate region 62a, the second intermediate region 62b, and the second end region 63, a layered state where the fiber-supplemented sheet 2 is layered on the elastic fiber sheet 1 is formed. With this layered state, although the fiber density of the elastic fiber sheet 1 is low, the fiber density is supplemented by the fiber-supplemented sheet 2. That is, when the fiber amount per unit area of the portion where the elastic fiber sheet 1 and the fiber-supplemented sheet 2 are integrally joined in a layered state becomes larger than the fiber amount per unit area of only the elastic fiber sheet 1, the fiber amount involved in joining in the portion increases. Since this sheet layered bodies 3 are superimposed in the sheet width direction, the fiber-supplemented sheet 2 is layered between the elastic fiber sheet 1 and the elastic fiber sheet 1 in a portion where the first end region 61 and the second end region 63 oppose each other and a portion where the first intermediate region 62a and the second intermediate region 62b oppose each other. Therefore, the fiber density is further supplemented by layering the fiber-supplemented sheets 2. In "(5) Side sealing portion formation step", portions that become formation targets of the side sealing portions 13 (the left-side sealing portion 13L and the right-side sealing portion 13R) of the sheet layered body 3 are heated and brought into a molten state in a state where the fiber density is supplemented by the fiber-supplemented sheet 2, whereby the opposing sheet layered bodies 3 are joined in the side sealing portions 13. Therefore, since the fiber amounts entangled in a molten state in the side sealing portion 13 can be secured, a sufficient joint strength in the side sealing portion 13 can be secured.

According to the method of manufacturing the wearing article 100, the layering region 60 is set so as to include the end in the side seal extending direction in the region where the side sealing portion 13 is formed, and the plurality of fiber-supplemented sheets 2 can be efficiently layered on the elastic fiber sheet 1 at both ends in the seal extending direction of both the side sealing portions 13L and 13R. Specifically, since the layering region 60 (the first end region 61 and the second end region 63) extending in the sheet longitudinal direction from one of the both ends of the left-side sealing portion 13L to one of the both ends of the right-side sealing portion 13R and the layering region 60 (the first intermediate region 62a and the second intermediate region 62b) extending in the sheet longitudinal direction from the other of the both ends of the left-side sealing portion 13L to the other of the both ends of the right-side sealing portion 13R are set, it is possible to efficiently dispose the fiber-supplemented sheets 2 at both ends of both the side sealing portions 13 by layering four fiber-supplemented sheets 2 in these four layering regions 60 of the elastic fiber sheet 1.

Furthermore, according to the above method of manufacturing, the sheet layered body 3 extending in the sheet longitudinal direction can be formed by layering the plurality of fiber-supplemented sheets 2 extending in the sheet longitudinal direction on the plurality of layering regions 60 of the elastic fiber sheet 1 extending in the sheet longitudinal direction. Thus, the plurality of wearing articles 100 can be efficiently mass-manufactured.

When the elastic fiber sheet 1 and the plurality of fiber-supplemented sheets 2 are conveyed in the same sheet longitudinal direction, the plurality of fiber-supplemented sheets 2 can be more efficiently layered on the elastic fiber sheet 1 along the conveyance flow of the elastic fiber sheet 1.

Furthermore, according to the method of manufacturing the wearing article, the elastic fiber sheet 1 can be reinforced by securing the thickness of the fiber-supplemented sheet 2 at the time of joining while reducing the thickness of the fiber-supplemented sheet 2 before joining.

Specifically, before joining, the fiber-supplemented sheet 2 is layered on the elastic fiber sheet 1 in a dispersed manner in each of the pair of opposable regions at the left-front end 11L and the left-rear end 12L. Before joining, the fiber-supplemented sheet 2 is disposed in a dispersed manner in each of the pair of opposable regions at the right-front end 11R and the right-rear end 12R. Such dispersion disposition makes it possible to layer the fiber-supplemented sheet 2 between the elastic fiber sheet 1 and the elastic fiber sheet 1 in the left-side sealing portion 13L in which the left-front end 11L and the left-rear end 12L are joined to oppose each other and the right-side sealing portion 13R in which the right-front end 11R and the right-rear end 12R are joined to oppose each other while reducing the thickness of the fiber-supplemented sheet 2 before joining. This can secure a large fiber amount that can be involved in joining, and thus it is possible to secure the joint strength of the left-side sealing portion 13L and the right-side sealing portion 13R.

In "(2) Second sheet preparation step", the elastic members 81 to 83 can be disposed when the elastic fiber sheet 1 and the fiber-supplemented sheet 2 are layered, and thus the elastic members 81 to 83 can be efficiently disposed as compared with a case where a sheet other than the fiber-supplemented sheet 2 is prepared for disposing the elastic members 81 to 83 and this is layered on the elastic fiber sheet 1.

Since the elastic members 81 to 83 are disposed over the entire length of the elastic fiber sheet 1 and the fiber-supplemented sheet 2 in the sheet longitudinal direction, the elastic members 81 to 83 can be efficiently disposed over the entire length in the article width direction of the wearing article 100 after manufacturing as compared with the case where the elastic members 81 to 83 are intermittently disposed in the sheet longitudinal direction. Since the elastic members 81 to 83 are elastic in the sheet longitudinal direction, it is possible to improve elasticity in the article width direction over the entire length in the article width direction of the wearing article 100 after manufacturing.

Note that the present invention is not limited to the above embodiment, and for example, the following aspects can also be adopted.

### (A) Region for securing joint strength

In the wearing article of the present invention, the setting place of the sheet layering region, the size of the region, and the like are not limited to those of the above embodiment.

### (A1) Setting place of sheet layering region

The setting place of the sheet layering region is not limited to the front waist region 51, the front crotch region 53, the rear waist region 52, and the rear crotch region 54 of the above embodiment. The sheet layering region may be set in at least a part of the left-side sealing portion and at least a part of the right-side sealing portion. For example, the sheet layering region may be set only in a region of the center in the left seal extending direction in the left-side sealing portion, may be set at both ends and the center in the left seal extending direction, or may be set in the entire region in the left seal extending direction. Similarly, the sheet layering region may be set only in a region of the center in the right seal extending direction in the right-side sealing portion, may be set in both the both ends and the center in the right seal extending direction, or may be set in the entire region in the right seal extending direction.

Furthermore, the present invention is not limited to the aspect in which the sheet layering region is set in both the left-front end and the left-rear end, and the sheet layering region may be set in at least one of the left-front end and the left-rear end. Similarly, the present invention is not limited to the aspect in which the sheet layering region is set in both the right-front end and the right-rear end, and the sheet layering region may be set in at least one of the right-front end and the right-rear end. Therefore, in the method of manufacturing illustrated in FIG. 6, the plurality of layering regions 60 need not necessarily include the pair of opposable regions.

### (A2) Shape of sheet layering region

As long as the sheet layering region is set in at least a part of the left-side sealing portion and at least a part of the right-side sealing portion, the shape of the sheet layering region is not limited to that of the above embodiment.

For example, in the above embodiment, the front crotch region 53 is a trapezoidal region positioned in the crotch on the front abdominal side of the wearer, but the front crotch region 53 may be only a region where the front crotch elastic member 33 is disposed. In the case of the above embodiment, the front crotch region 53 may be a region along the both front crotch inclined sides 11T and the front coupling side 11J. The front crotch region 53 may be a linear region extending in the article width direction from the left-front crotch end 11CL to the right-front crotch end 11CR. Similarly, the rear crotch region 54 may also be only a region where the rear crotch elastic member 34 is disposed. In the case of the above embodiment, the rear crotch region 54 may be a region along the both rear crotch inclined sides 12T and the rear coupling side 12J. The rear crotch region 54 may be a linear region extending in the article width direction from the left-rear crotch end 12CL to the right-rear crotch end 12CR.

### (B) Non-disposition region

In the present invention, air permeability may be secured by setting the non-disposition region in at least a part of the front waist part and the rear waist part, and the disposition place, size, and the like of the non-disposition region are not limited to those of the above embodiment. For example, the entire surface of any one of the front waist part 11 and the rear waist part 12 may be set as the non-disposition region.

### (C) Elastic member

### (C1) Omission of elastic member

In the present invention, the elastic member can be omitted. For example, when elasticity by the elastic member is not required in the wearing article, or when elasticity of the wearing article is secured by the elastic fiber sheet, the elastic member can be omitted. The elastic member can be omitted at any position. For example, in the above embodiment, at least one of the front waist elastic member, the rear waist elastic member, the front crotch elastic member, and the rear crotch elastic member can be omitted.

### (C2) Weakening treatment of elastic member

At least one of the front crotch elastic member and the rear crotch elastic member may be subjected to weakening treatment at a portion where the absorbent main body is disposed. The weakening treatment is treatment of weakening the elastic force of the elastic member or treatment of disabling the elastic force of the elastic member. The weakening treatment is performed, for example, by cutting, with a blade, the elastic member at the portion where the absorbent main body is positioned or fusing the elastic member with heat.

### (D) Aspect of wearing article

### (D1) Shapes of front waist part and rear waist part

The shapes of the front waist part and the rear waist part of the present invention are not limited as long as the front waist part covers the front abdominal part and the rear waist part covers the back part. For example, at least one of the front waist part and the rear waist part may have, for example, a rectangular shape.

### (D2) Configuration of crotch of wearing article

In the present invention, the wearing article may be configured to cover from the front abdominal part to the back part of the wearer via the crotch, and the form of the front waist part and the rear waist part at the crotch is not limited. For example, in the above embodiment, the front waist part 11 and the rear waist part 12 are separated from each other and coupled via the absorbent main body 20, but the front waist part and the rear waist part may be continuous at the crotch. When the front waist part and the rear waist part are continuous at the crotch, "(3) Separation step" can be omitted in the method of manufacturing illustrated in FIG. 6. That is, the sheet layered body may be continuous in the sheet width direction. Alternatively, in "(3) Separation step", the opening may be formed in the sheet layered body so as to have the left leg hole and the right leg hole in a state where the region where the front waist part is formed and the region where the rear waist part is formed are continuous in the portion corresponding to the crotch. Furthermore, the absorbent main body 20 may be omitted.

### (E) Left-side sealing portion and right-side sealing portion

### (E1) Method of joining

In the present invention, the left-side sealing portion and the right-side sealing portion may be joined, and the method of joining is not limited to the thermal welding of the above embodiment.

For example, the left-front end and the left-rear end may be joined by adhesion with an adhesive. Specifically, when the left-front end and the left-rear end of the present invention are superimposed, the fiber-supplemented sheet is interposed between the elastic fiber sheet and the elastic fiber sheet. When the fiber amount per unit area of the portion where the elastic fiber sheet and the fiber-supplemented sheet are integrally joined in a layered state becomes larger than the fiber amount per unit area of only the elastic fiber sheet, the fiber amount involved in joining in the portion increases, and thus the fiber amount with which the adhesive is entangled can be secured. Therefore, the joint strength of the left-side sealing portion can be secured. Similarly, in the right-side sealing portion, the right-front end and the right-rear end may be joined by adhesion with an adhesive.

When the left-side sealing portion and the right-side sealing portion are formed by an adhesive, the elastic fiber sheet and the fiber-supplemented sheet are not limited to a thermoplastic nonwoven fabric. As the adhesive, an epoxy resin, an acrylic resin, or the like can be adopted.

Other than that, after a thermoplastic nonwoven fabric is adopted for the elastic fiber sheet and the fiber-supplemented sheet, ultrasonic welding and thermal welding can be used for joining the left-side sealing portion and the right-side sealing portion.

### (E2) Formation timing of left-side sealing portion and right-side sealing portion

In the present invention, the formation timing of the left-side sealing portion and the right-side sealing portion is not limited to the same time as in the above embodiment, and may be formed in different formation steps. For example, the side sealing portion formation step includes the left-side sealing portion formation step of forming the left-side sealing portion and the right-side sealing portion formation step of forming the right-side sealing portion, and these formation steps may be performed at different timings.

For example, in the left-side sealing portion formation step, in a state where the sheet layered body is superimposed in the sheet width direction, a portion that becomes a formation target of the left-side sealing portion of the sheet layered body is heated at intervals for each portion where the wearing article is formed in the sheet longitudinal direction. Due to this, the left-side sealing portion of each wearing article is formed in the sheet layered body. In the right-side sealing portion formation step, in a state where the sheet layered body is superimposed in the sheet width direction, a portion that becomes a formation target of the right-side sealing portion of the sheet layered body is heated at intervals for each portion where the wearing article is formed in the sheet longitudinal direction. Due to this, the right-side sealing portion of each wearing article is formed in the sheet layered body.

### (F) Conveyance in method of manufacturing wearing article

In the method of manufacturing the wearing article of the present invention, each step may be performed without conveyance. For example, the fiber-supplemented sheet may be joined in a layered state on the elastic fiber sheet in a stationary state. Other than that, the side sealing portion formation step and the clipping step may also be performed without conveyance.

### (G) Timing of layer of elastic fiber sheet and fiber-supplemented sheet

In the side sealing portion formation step of the present invention, the side sealing portion may be formed in a layered state where the fiber-supplemented sheet is layered on the elastic fiber sheet, and the timing of forming the layered state is not limited. In the above embodiment, the fiber-supplemented sheet 2 is layered on the elastic fiber sheet 1 in the step of layering the fiber-supplemented sheet 2 on the elastic fiber sheet 1, that is, before the side sealing portion formation step. However, in the side sealing portion formation step, the fiber-supplemented sheet may be layered on the elastic fiber sheet at the timing when the left-side sealing portion and the right-side sealing portion are joined. Specifically, in the side sealing portion formation step, the left-side sealing portion and the right-side sealing portion may be formed in the sheet layered body simultaneously with sandwiching the fiber-supplemented sheet between the both elastic fiber sheets when the elastic fiber sheets are superimposed with each other.

### (H) Cutting step

### (H1) Timing of cutting step

The timing of the cutting step is not limited to the timing of the above embodiment.

In the above embodiment, the cutting step is performed after the side sealing portion formation step, but the side sealing portion formation step may be performed after the cutting step. Specifically, before forming the side sealing portion, the sheet layered body is cut so as to be divided in the sheet longitudinal direction for each portion forming the wearing article. The wearing article is formed by forming a left-side sealing portion and a right-side sealing portion in a state where the cut sheet layered bodies oppose each other in the sheet width direction.

The cutting step may be performed at an initial stage in the method of manufacturing the wearing article. For example, in the cutting step, the elastic fiber sheet is cut so as to be divided in the sheet longitudinal direction, thereby clipping a plurality of elastic fiber sheet division pieces. Each of the elastic fiber sheet division pieces includes a portion constituting the front waist part and a portion constituting the rear waist part arranged side by side in the sheet width direction. The elastic member and the fiber-supplemented sheet are layered in the plurality of sheet layering regions in the portion constituting the front waist part and the portion constituting the rear waist part. Thereafter, the left-side sealing portion and the right-side sealing portion are formed in a state where the elastic member and the fiber-supplemented sheet are layered, and in a state where the elastic fiber sheet division pieces oppose each other in the sheet width direction. Thus, the wearing article is formed.

### (H2) Aspect of cutting

In the embodiment described above, in the cutting step, the crotch of the front waist part and the crotch of the rear waist part are clipped from the sheet layered body in a state of being coupled by the absorbent main body. Different from this, in the cutting step, the front waist part and the rear waist part may be clipped from the sheet layered body in a state of being separated from each other. Then, in the side sealing formation step, the side sealing portion is formed in the front waist part and the rear waist part that are separated from each other. The crotch of the front waist part and the crotch of the rear waist part that are separated from each other are joined to each other or coupled to each other via the absorbent main body.

Note that the above-described specific embodiment mainly includes the invention having the following configuration.

A method of manufacturing a wearing article for solving the above problems is a method of manufacturing a wearing article that covers a front abdominal part, a crotch, and a back part of a wearer, the wearing article including a front waist part including a left-front end and a right-front end in an article width direction along a waist of the wearer and covering the front abdominal part of the wearer, and a rear waist part including a left-rear end and a right-rear end in the article width direction forming a left-side sealing portion and a right-side sealing portion, respectively, by being joined to an inner side surface in a state of being layered on the inner side surface of each of the left-front end and the right-front end and covering the back part of the wearer, the rear waist part being coupled to the front waist part at a portion positioned in the crotch of the wearer, the method including: a preparation step of preparing an elastic fiber sheet formed of a nonwoven fabric including an elastic fiber formed of a fiber having elasticity, and an extensible stretching fiber having elasticity smaller than the elasticity of the elastic fiber, the nonwoven fabric subjected to drawing processing; a left-side sealing portion formation step of forming the left-side sealing portion by superimposing and joining the left-front end and the left-rear end of each wearing article in a layered state where a fiber-supplemented sheet formed of a nonwoven fabric is layered on the elastic fiber sheet in at least a part of a portion that is a formation target of the left-side sealing portion; a right-side sealing portion formation step of forming the right-side sealing portion by superimposing and joining the right-front end and the right-rear end of each wearing article in the layered state in at least a part of a portion that is a formation target of the right-side sealing portion; and a clipping step of clipping the front waist part and the rear waist part from the elastic fiber sheet, in which the fiber-supplemented sheet is not layered on the elastic fiber sheet in at least a part of the elastic fiber sheet after the left-side sealing portion formation step and the right-side sealing portion formation step, and airflow through the elastic fiber sheet is allowed.

According to the wearing article manufactured by the method of manufacturing the wearing article, the joint strength of the left-side sealing portion and the right-side sealing portion can be secured although the fiber density of the elastic fiber sheet is low, and air permeability of the elastic fiber sheet can be secured.

Specifically, in the left-side sealing portion, the elastic fiber sheet and the elastic fiber sheet are joined in a layered state where the fiber-supplemented sheet is interposed between the elastic fiber sheet and the elastic fiber sheet, so that the fiber density can be supplemented by the fiber-supplemented sheet although the fiber density of the elastic fiber sheet is low, whereby a sufficient joint strength can be secured. The fiber amount per unit area of the portion where the elastic fiber sheet and the fiber-supplemented sheet are integrally joined in a layered state becomes larger than the fiber amount per unit area of only the elastic fiber sheet. Thereby, the fiber amount involved in joining in the portion increases, and thus a sufficient joint strength can be secured. Similarly, in the right-side sealing portion, the fiber density can be supplemented by interposing the fiber-supplemented sheet between the elastic fiber sheets, whereby a sufficient joint strength can be secured.

On the other hand, since the fiber-supplemented sheet is not layered on the elastic fiber sheet in at least a part of the elastic fiber sheet, airflow through the elastic fiber sheet is secured.

In the method of manufacturing the wearing article, preferably, the left-side sealing portion formation step includes superimposing and joining the left-front end and the left-rear end in the layered state at least at both ends in a left seal extending direction in a portion that is a formation target of the left-side sealing portion, and the left seal extending direction is a direction orthogonal to the article width direction and a direction where the left-side sealing portion extends, and the right-side sealing portion formation step includes superimposing and joining the right-front end and the right-rear end in the layered state at least at both ends in a right seal extending direction in a portion that is a formation target of the right-side sealing portion, and the right seal extending direction is a direction orthogonal to the article width direction and a direction where the right-side sealing portion extends.

According to the wearing article manufactured by the method of manufacturing the wearing article, since the joint strength can be secured at both ends in the direction where the left-side sealing portion and the right-side sealing portion (hereinafter, the left-side sealing portion and the right-side sealing portion are collectively referred to as side sealing portions) extend, it is possible to suppress the side sealing portion from being unintentionally torn from the both ends in the extending direction.

Specifically, the fiber-supplemented sheet is layered on the elastic fiber sheet at least at both ends in the left seal extending direction of the left-side sealing portion, whereby the joint strength at the both ends in the left seal extending direction is secured. The both ends are portions where stress concentration particularly occurs when the front waist part and the rear waist part in a state of being joined to each other are expanded in a direction away from each other. By ensuring the joint strength of the both ends as described above, it is possible to suppress the left-side sealing portion from being unintentionally torn from the both ends.

Similarly, since the joint strength of both ends of the right-side sealing portion in the right seal extending direction is secured, it is possible to suppress the right-side sealing portion from being unintentionally torn from the both ends, which are portions where stress concentration particularly occurs.

In the method of manufacturing the wearing article, preferably, the preparation step includes preparing an elastic fiber sheet extending in a sheet longitudinal direction corresponding to the article width direction as the elastic fiber sheet, and forming a sheet layered body in which a plurality of fiber-supplemented sheets extending in the sheet longitudinal direction is layered only in a plurality of sheet layering regions set to be arranged side by side at intervals in a sheet width direction orthogonal to the sheet longitudinal direction in the elastic fiber sheet.

According to the method of manufacturing the wearing article, the sheet layered body extending in the sheet longitudinal direction can be formed by layering the plurality of fiber-supplemented sheets extending in the sheet longitudinal direction in the plurality of sheet layering regions of the elastic fiber sheet extending in the sheet longitudinal direction. Thus, the plurality of wearing articles can be efficiently mass-manufactured.

For example, when the sheet layering region is set so as to include the end in the side seal extending direction in the region where the side sealing portion is formed, the plurality of fiber-supplemented sheets can be efficiently layered on the elastic fiber sheet at both ends in the side seal extending direction of both side sealing portions. Specifically, in a case of setting the sheet layering region extending in the sheet longitudinal direction from one of the both ends of the left-side sealing portion to one of the both ends of the right-side sealing portion and the sheet layering region extending in the sheet longitudinal direction from the other of the both ends of the left-side sealing portion to the other of the both ends of the right-side sealing portion, it is possible to efficiently dispose the fiber-supplemented sheets at both ends of the both side sealing portions by layering two fiber-supplemented sheets in these two sheet layering regions of the elastic fiber sheet.

In the method of manufacturing the wearing article, preferably, in the preparation step, the sheet layered body is formed by layering the plurality of fiber-supplemented sheets on the elastic fiber sheet while conveying both the elastic fiber sheet and the plurality of fiber-supplemented sheets in the sheet longitudinal direction.

According to the method of manufacturing the wearing article, by conveying the elastic fiber sheet and the plurality of fiber-supplemented sheets in the same direction, the plurality of fiber-supplemented sheets can be efficiently layered on the elastic fiber sheet along the flow of conveyance of the elastic fiber sheet.

In the method of manufacturing the wearing article, preferably, the plurality of sheet layering regions set in the preparation step include at least a pair of opposable regions that is opposable to each other in each of the front waist part and the rear waist part in the left-side sealing portion formation step and the right-side sealing portion formation step, in the left-side sealing portion formation step, the left-side sealing portion is formed in a state where the fiber-supplemented sheets disposed in the pair of opposable regions are layered between the elastic fiber sheet and the elastic fiber sheet, and in the right-side sealing portion formation step, the right-side sealing portion is formed in a state where the fiber-supplemented sheets disposed in the pair of opposable regions are layered between the elastic fiber sheet and the elastic fiber sheet.

According to the method of manufacturing the wearing article, the elastic fiber sheet can be reinforced by securing the thickness of the fiber-supplemented sheet at the time of joining while reducing the thickness of the fiber-supplemented sheet before joining.

Specifically, before joining, the fiber-supplemented sheet is layered on the elastic fiber sheet in a dispersed manner in each of the pair of opposable regions at the left-front end and the left-rear end. Before joining, the fiber-supplemented sheet is disposed in a dispersed manner in each of the pair of opposable regions at the right-front end and the right-rear end. Such dispersion disposition makes it possible to layer the fiber-supplemented sheet between the elastic fiber sheet and the elastic fiber sheet in the left-side sealing portion in which the left-front end and the left-rear end are joined to oppose each other and the right-side sealing portion in which the right-front end and the right-rear end are joined to oppose each other while reducing the thickness of the fiber-supplemented sheet before joining. This can secure a large fiber amount that can be involved in joining, and thus it is possible to secure the joint strength of the left-side sealing portion and the right-side sealing portion.

In the method of manufacturing the wearing article, preferably, in the preparation step, the fiber-supplemented sheet and the elastic fiber sheet are layered while an elastic member is sandwiched between the elastic fiber sheet and the fiber-supplemented sheet.

According to the method of manufacturing the wearing article, since the elastic member can be disposed when the elastic fiber sheet and the fiber-supplemented sheet are layered, the elastic member can be efficiently disposed as compared with a case where a sheet other than the fiber-supplemented sheet is prepared for disposing the elastic member and this is layered on the elastic fiber sheet.

In the method of manufacturing the wearing article, preferably, in the preparation step, the fiber-supplemented sheet is layered on the elastic fiber sheet over an entire length in the article width direction of at least one of the front waist part and the rear waist part.

According to the wearing article manufactured by the method of manufacturing the wearing article, the elastic member can be disposed using a space between the fiber-supplemented sheet and the elastic fiber sheet over the entire length in the article width direction. Therefore, the elastic member can be efficiently disposed over the entire length in the article width direction as compared with the case where the elastic member is intermittently disposed in the article width direction.

In the method of manufacturing the wearing article, preferably, in the preparation step, the elastic member is sandwiched between the elastic fiber sheet and the fiber-supplemented sheet to extend in the article width direction of the fiber-supplemented sheet and to be elastic in the article width direction.

According to the wearing article manufactured by the method of manufacturing the wearing article, elasticity can be imparted in the article width direction of the wearing article.

In the method of manufacturing the wearing article, preferably, the elastic fiber sheet and the fiber-supplemented sheet are thermoplastic nonwoven fabrics, and in the left-side sealing portion formation step and the right-side sealing portion formation step, the left-side sealing portion and the right-side sealing portion are formed by thermally welding the elastic fiber sheet and the fiber-supplemented sheet by heating a portion that becomes a formation target of the left-side sealing portion and a portion that becomes a formation target of the right-side sealing portion.

According to the method of manufacturing the wearing article, it is possible to form the both side sealing portions in a state where the fiber amount is increased by the fiber-supplemented sheet. Therefore, the joint strength of the both side sealing portions can be secured. Note that the "thermal welding" is not limited to a case where heat is directly applied to a sheet, and includes a case where the sheet is heated by applying ultrasonic vibration to the sheet.

A wearing article for solving the above problem is a wearing article that covers a front abdominal part, a crotch, and a back part of a wearer, the wearing article including: a front waist part including a left-front end and a right-front end in an article width direction along a waist of the wearer and covering the front abdominal part of the wearer; and a rear waist part including a left-rear end and a right-rear end in the article width direction forming a left-side sealing portion and a right-side sealing portion, respectively, by being joined to an inner side surface in a state of being layered on the inner side surface of each of the left-front end and the right-front end and covering the back part of the wearer, the rear waist part being coupled to the front waist part at a portion positioned in the crotch of the wearer, in which the front waist part and the rear waist part include an elastic fiber sheet formed of a nonwoven fabric including an elastic fiber formed of a fiber having elasticity, and an extensible stretching fiber having elasticity smaller than the elasticity of the elastic fiber, the nonwoven fabric subjected to drawing processing, in the left-side sealing portion, in at least a part of the left-side sealing portion, the left-front end and the left-rear end of each wearing article are superimposed and joined in a layered state where a fiber-supplemented sheet formed of a nonwoven fabric is layered on the elastic fiber sheet, in the right-side sealing portion, in at least a part of the right-side sealing portion, the right-front end and the right-rear end of each wearing article are superimposed and joined in the layered state, and the fiber-supplemented sheet is not layered on the elastic fiber sheet in at least a part of the elastic fiber sheet, and airflow through the elastic fiber sheet is allowed.

According to the wearing article, the joint strength of the left-side sealing portion and the right-side sealing portion can be secured although the fiber density of the elastic fiber sheet is low, and air permeability of the elastic fiber sheet can be secured.

Specifically, in the left-side sealing portion, since the elastic fiber sheet and the elastic fiber sheet are joined in a layered state where the fiber-supplemented sheet is interposed between the elastic fiber sheet and the elastic fiber sheet, the fiber density can be supplemented by the fiber-supplemented sheet although the fiber density of the elastic fiber sheet is low, whereby a sufficient joint strength can be secured. The fiber amount per unit area of the portion where the elastic fiber sheet and the fiber-supplemented sheet are integrally joined in a layered state becomes larger than the fiber amount per unit area of only the elastic fiber sheet. Thereby, the fiber amount involved in joining in the portion increases, and thus a sufficient joint strength can be secured. Similarly, in the right-side sealing portion, the fiber density can be supplemented by the fiber-supplemented sheet interposed between the elastic fiber sheets, whereby a sufficient joint strength can be secured.

On the other hand, since the fiber-supplemented sheet is not layered on the elastic fiber sheet in at least a part of the elastic fiber sheet, airflow through the elastic fiber sheet is secured.

In the wearing article, preferably, the left-front end and the left-rear end are superimposed and joined in the layered state at least at both ends in a left seal extending direction of the left-side sealing portion, the right-front end and the right-rear end are superimposed and joined in the layered state at least at both ends in a right seal extending direction of the right-side sealing portion, and the left seal extending direction is a direction orthogonal to the article width direction and is a direction where the left-side sealing portion extends, and the right seal extending direction is a direction orthogonal to the article width direction and is a direction where the right-side sealing portion extends.

According to the wearing article, since the joint strength can be secured at both ends in the direction where the left-side sealing portion and the right-side sealing portion extend, it is possible to suppress the side sealing portion from being unintentionally torn from the both ends in the extending direction.

The wearing article preferably further includes an elastic member disposed between the elastic fiber sheet and the fiber-supplemented sheet.

According to the wearing article, the elasticity of the portion can be improved by including the elastic member provided in the portion where the fiber-supplemented sheet is layered on the elastic fiber sheet.

In the wearing article, preferably, the fiber-supplemented sheet is disposed over an entire length in the article width direction of at least one of the front waist part and the rear waist part.

According to the wearing article, since the elastic member is disposed over the entire length in the article width direction of at least one of the front waist part and the rear waist part, the elasticity over the entire length in the article width direction of the wearing article can be improved as compared with the case where the elastic member is intermittently disposed in the article width direction.

In the wearing article, preferably, the elastic member extends in the article width direction of the fiber-supplemented sheet and is elastic in the article width direction.

According to the wearing article, the elastic member can improve the elasticity in the article width direction of the wearing article.

In the wearing article, preferably, the elastic fiber sheet and the fiber-supplemented sheet are thermoplastic nonwoven fabrics, and the left-front end and the left-rear end are thermally welded, and the right-front end and the right-rear end are thermally welded.

According to the wearing article, since the both side sealing portions are formed in a state where the fiber amount is increased by the fiber-supplemented sheet, the joint strength of the both side sealing portions can be secured. Note that the "thermal welding" is not limited to a case where heat is directly applied to a sheet, and includes a case where the sheet is heated by applying ultrasonic vibration to the sheet.

In the wearing article, preferably, the fiber density of the fiber-supplemented sheet is larger than the fiber density of the elastic fiber sheet.

According to the wearing article, the fiber amount per unit area of the portion where the elastic fiber sheet and the fiber-supplemented sheet are integrally joined in a layered state becomes reliably larger than the fiber amount per unit area of only the elastic fiber sheet, whereby a sufficient joint strength in the portion can be secured.

## Claims

1. A method of manufacturing a wearing article that covers a front abdominal part, a crotch, and a back part of a wearer, the wearing article including a front waist part including a left-front end and a right-front end in an article width direction along a waist of the wearer and covering the front abdominal part of the wearer, and a rear waist part including a left-rear end and a right-rear end in the article width direction forming a left-side sealing portion and a right-side sealing portion, respectively, by being joined to an inner side surface in a state of being layered on the inner side surface of each of the left-front end and the right-front end and covering the back part of the wearer, the rear waist part being coupled to the front waist part at a portion positioned in the crotch of the wearer, the method comprising:
a preparation step of preparing an elastic fiber sheet formed of a nonwoven fabric including an elastic fiber formed of a fiber having elasticity, and an extensible stretching fiber having elasticity smaller than the elasticity of the elastic fiber, the nonwoven fabric subjected to drawing processing;
a left-side sealing portion formation step of forming the left-side sealing portion by superimposing and joining the left-front end and the left-rear end of each wearing article in a layered state where a fiber-supplemented sheet formed of a nonwoven fabric is layered on the elastic fiber sheet in at least a part of a portion that is a formation target of the left-side sealing portion;
a right-side sealing portion formation step of forming the right-side sealing portion by superimposing and joining the right-front end and the right-rear end of each wearing article in the layered state in at least a part of a portion that is a formation target of the right-side sealing portion; and
a clipping step of clipping the front waist part and the rear waist part from the elastic fiber sheet,
wherein the fiber-supplemented sheet is not layered on the elastic fiber sheet in at least a part of the elastic fiber sheet after the left-side sealing portion formation step and the right-side sealing portion formation step, and airflow through the elastic fiber sheet is allowed.

2. The method of manufacturing the wearing article according to claim 1, wherein
the left-side sealing portion formation step includes superimposing and joining the left-front end and the left-rear end in the layered state at least at both ends in a left seal extending direction in a portion that is a formation target of the left-side sealing portion, and the left seal extending direction is a direction orthogonal to the article width direction and a direction where the left-side sealing portion extends, and
the right-side sealing portion formation step includes superimposing and joining the right-front end and the right-rear end in the layered state at least at both ends in a right seal extending direction in a portion that is a formation target of the right-side sealing portion, and the right seal extending direction is a direction orthogonal to the article width direction and a direction where the right-side sealing portion extends.

3. The method of manufacturing the wearing article according to claim 1 or 2, wherein
the preparation step includes preparing an elastic fiber sheet extending in a sheet longitudinal direction corresponding to the article width direction as the elastic fiber sheet, and forming a sheet layered body in which a plurality of fiber-supplemented sheets extending in the sheet longitudinal direction is layered only in a plurality of sheet layering regions set to be arranged side by side at intervals in a sheet width direction orthogonal to the sheet longitudinal direction in the elastic fiber sheet.

4. The method of manufacturing the wearing article according to claim 3, wherein
in the preparation step, the sheet layered body is formed by layering the plurality of fiber-supplemented sheets on the elastic fiber sheet while conveying both the elastic fiber sheet and the plurality of fiber-supplemented sheets in the sheet longitudinal direction.

5. The method of manufacturing the wearing article according to claim 3 or 4, wherein
the plurality of sheet layering regions set in the preparation step include at least a pair of opposable regions that is opposable to each other in each of the front waist part and the rear waist part in the left-side sealing portion formation step and the right-side sealing portion formation step,
in the left-side sealing portion formation step, the left-side sealing portion is formed in a state where the fiber-supplemented sheets disposed in the pair of opposable regions are layered between the elastic fiber sheet and the elastic fiber sheet, and
in the right-side sealing portion formation step, the right-side sealing portion is formed in a state where the fiber-supplemented sheets disposed in the pair of opposable regions are layered between the elastic fiber sheet and the elastic fiber sheet.

6. The method of manufacturing the wearing article according to any one of claims 1 to 5, wherein
in the preparation step, the fiber-supplemented sheet and the elastic fiber sheet are layered while an elastic member is sandwiched between the elastic fiber sheet and the fiber-supplemented sheet.

7. The method of manufacturing the wearing article according to claim 6, wherein
in the preparation step, the fiber-supplemented sheet is layered on the elastic fiber sheet over an entire length in the article width direction of at least one of the front waist part and the rear waist part.

8. The method of manufacturing the wearing article according to claim 7, wherein
in the preparation step, the elastic member is sandwiched between the elastic fiber sheet and the fiber-supplemented sheet to extend in the article width direction of the fiber-supplemented sheet and to be elastic in the article width direction.

9. The method of manufacturing the wearing article according to any one of claims 1 to 8, wherein
the elastic fiber sheet and the fiber-supplemented sheet are thermoplastic nonwoven fabrics, and
in the left-side sealing portion formation step and the right-side sealing portion formation step, the left-side sealing portion and the right-side sealing portion are formed by thermally welding the elastic fiber sheet and the fiber-supplemented sheet by heating a portion that becomes a formation target of the left-side sealing portion and a portion that becomes a formation target of the right-side sealing portion.

10. A wearing article that covers a front abdominal part, a crotch, and a back part of a wearer, the wearing article comprising:
a front waist part including a left-front end and a right-front end in an article width direction along a waist of the wearer and covering the front abdominal part of the wearer; and
a rear waist part including a left-rear end and a right-rear end in the article width direction forming a left-side sealing portion and a right-side sealing portion, respectively, by being joined to an inner side surface in a state of being layered on the inner side surface of each of the left-front end and the right-front end and covering the back part of the wearer, the rear waist part being coupled to the front waist part at a portion positioned in the crotch of the wearer,
wherein the front waist part and the rear waist part include an elastic fiber sheet formed of a nonwoven fabric including an elastic fiber formed of a fiber having elasticity, and an extensible stretching fiber having elasticity smaller than the elasticity of the elastic fiber, the nonwoven fabric subjected to drawing processing,
in the left-side sealing portion, in at least a part of the left-side sealing portion, the left-front end and the left-rear end of each wearing article are superimposed and joined in a layered state where a fiber-supplemented sheet formed of a nonwoven fabric is layered on the elastic fiber sheet,
in the right-side sealing portion, in at least a part of the right-side sealing portion, the right-front end and the right-rear end of each wearing article are superimposed and joined in the layered state, and
the fiber-supplemented sheet is not layered on the elastic fiber sheet in at least a part of the elastic fiber sheet, and airflow through the elastic fiber sheet is allowed.

11. The wearing article according to claim 10, wherein
the left-front end and the left-rear end are superimposed and joined in the layered state at least at both ends in a left seal extending direction of the left-side sealing portion,
the right-front end and the right-rear end are superimposed and joined in the layered state at least at both ends in a right seal extending direction of the right-side sealing portion, and
the left seal extending direction is a direction orthogonal to the article width direction and is a direction where the left-side sealing portion extends, and the right seal extending direction is a direction orthogonal to the article width direction and is a direction where the right-side sealing portion extends.

12. The wearing article according to claim 10 or 11, further comprising:
an elastic member disposed between the elastic fiber sheet and the fiber-supplemented sheet.

13. The wearing article according to claim 12, wherein
the fiber-supplemented sheet is disposed over an entire length in the article width direction of at least one of the front waist part and the rear waist part.

14. The wearing article according to claim 13, wherein
the elastic member extends in the article width direction of the fiber-supplemented sheet and is elastic in the article width direction.

15. The wearing article according to any one of claims 10 to 14, wherein
the elastic fiber sheet and the fiber-supplemented sheet are thermoplastic nonwoven fabrics, and
the left-front end and the left-rear end are thermally welded, and the right-front end and the right-rear end are thermally welded.

16. The wearing article according to any one of claims 10 to 15, wherein
a fiber density of the fiber-supplemented sheet is larger than a fiber density of the elastic fiber sheet.
